(19) Europäisches Patentamt
European Patent Office
Office européen des brevets

(11) Publication number: **0 430 485 A2**

# (12) EUROPEAN PATENT APPLICATION

(21) Application number: **90312392.5**

(22) Date of filing: **13.11.90**

(51) Int. Cl.[5]: **C07D 471/04,** // A61K31/435, A61K31/55, (C07D471/04, 221:00, 221:00), (C07D471/04, 221:00, 209:00), (C07D471/04, 223:00, 221:00)

(30) Priority: **29.11.89 JP 307591/89**

(43) Date of publication of application: **05.06.91 Bulletin 91/23**

(84) Designated Contracting States: **DE FR GB**

(71) Applicant: **UBE INDUSTRIES, LTD.
12-32, Nishihonmachi 1-chome
Ube-shi, Yamaguchi-ken (JP)**

(72) Inventor: **Kuroki, Yoshiaki, c/o Ube Res. Lab. Ube Ind. Ltd.
1978-5, Oaza Kogushi, Ube-shi
Yamaguchi-ken (JP)**

Inventor: **Fujiwara, Hiroshi, c/o Ube Res. Lab. Ube Ind. Ltd.
1978-5, Oaza Kogushi, Ube-shi
Yamaguchi-ken (JP)**

Inventor: **Nishino, Shigeyoshi, c/o Ube Res. Lab. Ube Ind.
Ltd., 1978-5, Oaza Kogushi, Ube-shi
Yamaguchi-ken (JP)**

Inventor: **Nakamura, Izumi, c/o Ube Res. Lab. Ube Ind.
Ltd., 1978-5, Oaza Kogushi, Ube-shi
Yamaguchi-ken (JP)**

Inventor: **Tokunaga, Haruko, c/o Ube Res. Lab. Ube Ind.
Ltd., 1978-5, Oaza Kogushi, Ube-shi
Yamaguchi-ken (JP)**

(74) Representative: **Green, Mark Charles et al
Urquhart-Dykes & Lord, 91 Wimpole Street
London W1M 8AH (GB)**

## (54) Quinoline compound.

(57) There is disclosed a quinoline compound represented by the following formula (I):

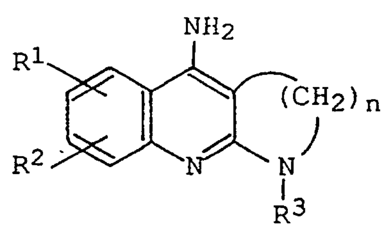

(I)

wherein $R^1$ and $R^2$ each represent a hydrogen atom, a halogen atom, a lower alkyl group, a trifluoromethyl group, a hydroxyl group, a lower alkoxy group, a lower alkanoyloxy group, a nitro group, an amino group or a lower alkanoylamino group; $R^3$ represents a hydrogen atom, an alkyl group having 1 to 15 carbon atoms, a cycloalkyl group, an aralkyl group having 7 to 15 carbon atoms which may be substituted by a halogen atom, a lower alkyl group or a lower alkoxy group, an alkanoyl group having 2 to 15 carbon atoms, or a benzoyl group which may be substituted by a halogen atom, a lower alkyl group, a lower alkoxy group, a nitro group, a hydroxy group or an amino group; and n represents an integer of 2 to 5,
and an acid addition salt thereof.

EP 0 430 485 A2

# QUINOLINE COMPOUND

## BACKGROUND OF THE INVENTION

This invention relates to novel quinolines compounds and acid addition salts thereof having acetylcholine esterase inhibiting activity which are useful as a memory disorder therapeutic agent for senile dementia or Alzheimer's disease.

With increase of old aged people, senile dementia and Alzheimer's disease which cause memory disorder as a main symptom have become a serious social problem.

According to the research in recent years, it has been clarified that the central cholinergic neurons deeply participates in learning and memory processes, and acetylcholine content decreases in the brains of the patients with senile dementia and Alzheimer's disease.

The acetylcholine esterase inhibiting agent has been expected to become a useful preventive and therapeutic agent for senile dementia and Alzheimer's disease, because it increases the amount of acetylcholine in brain. For example, by using Physostigmine (Neurology, vol. 8, p. 397 (1978)) and 9-amino-1,2,3,4-tetrahydroacridine (Tacline) (The New England Journal of Medicine, vol. 315, p. 1241 (1986)) which are the representatives of acetylcholine esterase inhibiting agents, have been investigated as therapeutic agents of senile dementia and Alzheimer's disease.

Many acetylcholine esterase inhibiting agents have been already reported, however, they have serious side effect against the peripheral nervous system, and have short effect sustaining duration, or have high toxicity. Therefore, they are not necessarily sufficient to be applied to human.

## SUMMARY OF THE INVENTION

An object of the present invention is to provide a novel compound which has high inhibitory effect on acetylcholine esterase and has the high safety and the excellent central selectivity, i.e. is useful as a preventive and therapeutic agent with less side effect for senile dementia and Alzheimer's disease.

The present inventors have made various investigation and as the results, they have found that a novel quinoline derivative represented by the formula (I) has an excellent acetylcholine esterase inhibiting activity and less side effect, whereby it is extremely useful as a therapeutic agent for senile dementia.

That is, the present invention relates to a quinoline compound represented by the following formula (I) :

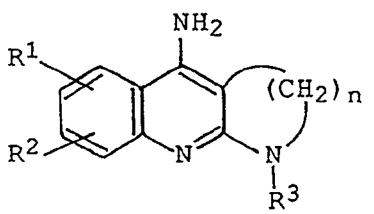

(I)

wherein $R^1$ and $R^2$ each represent a hydrogen atom, a halogen atom, a lower alkyl group, a trifluoromethyl group, a hydroxyl group, a lower alkoxy group, a lower alkanoyloxy group, a nitro group, an amino group or a lower alkanoylamino group ; $R^3$ represents a hydrogen atom, an alkyl group having 1 to 15 carbon atoms, a cycloalkyl group, an aralkyl group having 7 to 15 carbon atoms which may be substituted by a halogen atom, a lower alkyl group or a lower alkoxy group, an alkanoyl group having 2 to 15 carbon atoms, or a benzoyl group which may be substituted by a halogen atom, a lower alkyl group, a lower alkoxy group, a nitro group, a hydroxy group or an amino group ; and n represents an integer of 2 to 5,
and an acid addition salt thereof.

## DESCRIPTION OF THE PREFERRED EMBODIMENTS

Each group represented by each symbol in the formula (I) means the following meanings.

The halogen atom means a fluorine atom, a chlorine atom, a bromine atom and an iodine atom.

Each of the lower alkyl group, the lower alkoxy group, the lower alkanoyloxy group and the lower alkanoylamino group has 1 to 4 carbon atoms, and there may be mentioned, for example, a straight or branched alkyl group such as a methyl group, an ethyl group, a n-propyl group, an isopropyl group, a n-butyl group, a sec-butyl group, an isobutyl group and a t-butyl group ; an alkoxy group such as a methoxy group, an ethoxy

group, a n-propoxy group, an isopropoxy group, a n-butoxy group, a sec-butoxy group and an isobutoxy group; an alkanoyloxy group such as an acetoxy group, a n-propanoyloxy group, a n-butanoyloxy group and an isobutanoyloxy group ; and an alkanoylamino group such as an acetylamino group, a n-propanoylamino group, a n-butanoylamino group and an isobutanoylamino group.

As the alkyl group having 1 to 15 carbon atoms, there may be mentioned a straight or branched alkyl group such as a methyl group, an ethyl group, a n-propyl group, an isopropyl group, a n-butyl group, an isobutyl group, a sec-butyl group, a n-pentyl group, an isopentyl group, a neopentyl group, a hexyl group, a heptyl group, an octyl group, a decyl group, a dodecyl group and a pentadecyl group.

As the cycloalkyl group, there may be mentioned a cycloalkyl group having 3 to 10 carbon atoms such as a cyclopropyl group, a cyclobutyl group, a cyclopentyl group, a cyclohexyl group, a cycloheptyl group, a cyclooctyl group and a cyclodecyl group.

As the aralkyl group having 7 to 15 carbon atoms which may be substituted by a lower alkyl group or a lower alkoxy group, there may be mentioned a benzyl group, a 4-chlorobenzyl group, a 3-chlorobenzyl group, a 4-mehtoxybenzyl group, a 3-methoxybenzyl group, a 3,4-dimethoxybenzyl group, a 4-ethoxybenzyl group, a 4-methylbenzyl group, a 2-phenylethyl group, a 2-(4-methoxyphenyl) ethyl group, a 2-(4-fluorophenyl) ethyl group, a 2-(4-methylphenyl) ethyl group, a 3-phenylpropyl group, a 3-(4-fluorophenyl) propyl group, a 4-phenylbutyl group and a 4-(4-methoxyphenyl) butyl group.

Also, as the alkanoyl group having 2 to 15 carbon atoms, there may be mentioned a straight or branched alkanoyl group such as an acetyl group, a n-propanoyl group, a n-butanoyl group, an isobutanoyl group, a n-pentanoyl group, an isopentanoyl group, a n-hexanoyl group, a n-heptanoyl group, a n-octanoyl group, a n-decanoyl group and a n-dodecanoyl group.

As the benzoyl group which may be substituted by a halogen atom, a lower alkyl group, a lower alkoxy group, a nitro group, a hydroxy group or an amino group, there may be mentioned a benzoyl group, a 4-chlorobenzoyl group, a 3-chlorobenzoyl group, an 2-chlorobenzoyl group, a 4-methylbenzoyl group, a 4-methoxybenzoyl group, a 3-methoxybenzoyl group, a 4-nitrobenzoyl group, a 4-aminobenzoyl group and a 4-hydroxybenzoyl group. Said substituent(s) in the above aralkyl group and benzoyl group may be present one or more on the phenyl ring, and the substituted position is not particularly limited.

As the acid addition salt of the compound represented by the formula (I), there may include an inorganic acid addition salt or an organic acid addition salt, preferably a pharmaceutically acceptable acid addition salt.

As the inorganic acid addition salt, there may be mentioned a salt with hydrochloric acid, hydrobromic acid, sulfuric acid or phosphoric acid, and as the organic acid addition salt, there may be mentioned a salt with maleic acid, fumaric acid, oxalic acid or paratoluenesulfonic acid.

In the formula (I), the following quinoline compound and its acid addition salt are particularly preferred.

(1) A compound wherein n is 2 to 4.

(2) A compound wherein n is 2 and $R^3$ is a hydrogen atom, an alkyl group having 1 to 15 carbon atoms, a cycloalkyl group, or an aralkyl group having 7 to 15 carbon atoms which may be substituted by a halogen atom, a lower alkyl group or a lower alkoxy group.

(3) A compound wherein n is 2 and $R^3$ is an alkanoyl group having 2 to 15 carbon atoms, or a benzyl group which may be substituted by a halogen atom, a lower alkyl group or a lower alkoxy group.

(4) A compound wherein n is 3 and $R^3$ is a hydrogen atom, an alkyl group having 1 to 15 carbon atoms, a cycloalkyl group, or an aralkyl group having 7 to 15 carbon atoms which may be substituted by a halogen atom, a lower alkyl group or a lower alkoxy group.

(5) A compound wherein n is 3 and $R^3$ is an alkanoyl group having 2 to 15 carbon atoms, or a benzyl group which may be substituted by a halogen atom, a lower alkyl group or a lower alkoxy group.

(6) A compound wherein n is 4 and $R^3$ is a hydrogen atom, an alkyl group having 1 to 15 carbon atoms, a cycloalkyl group, or an aralkyl group having 7 to 15 carbon atoms which may be substituted by a halogen atom, a lower alkyl group or a lower alkoxy group.

(7) A compound wherein n is 4 and $R^3$ is an alkanoyl group having 2 to 15 carbon atoms, or a benzyl group which may be substituted by a halogen atom, a lower alkyl group or a lower alkoxy group.

In the formula (I) of the present invention, a compound (Ia) wherein $R^3$ represents an alkyl group or an aralkyl group can be prepared by cyclization of an amidine derivative (II) as shown below.

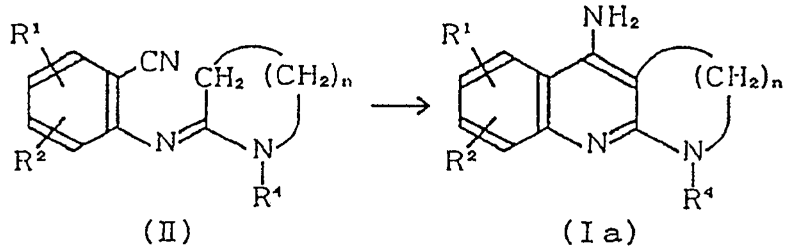

(II) (Ia)

wherein $R^4$ represents an alkyl group or an aralkyl group and $R^1$, $R^2$ and n have the same meanings as defined above.

The said cyclization reaction is preferably and generally carried out in the presence of an acid or a base. As the acid, there may be preferably used a Lewis acid such as zinc chloride, zinc bromide, aluminum chloride, aluminum bromide, tin tetrachloride, titanium tetrachloride and boron trifluoride etherate. Also, polyphosphoric acid may be used.

A reaction solvent is not particularly limited so long as it does not participate the reaction, but preferably includes an aromatic solvent such as nitrobenzene, chlorobenzene, toluene and xylene, and an amide type solvent such as dimethylacetamide and N-methylpyrrolidone. A reaction temperature is generally 50°C to 250°C, preferably 100°C to 200°C.

On the other hand, as the base, there may be preferably mentioned lithium diisopropylamide, butyl lithium, potassium butoxide, sodium ethoxide and sodium methoxide. In this case, as the reaction solvent, preferred are ether series solvents such as ether, tetrahydrofuran and dimethoxyethane ; and hydrocarbon series solvent such as benzene and hexane. A reaction temperature is optionally selected from the range of -80°C to 100°C.

Also, in the formula (I), a compound wherein $R^3$ is a hydrogen atom can be prepared, as shown in the following reaction scheme, by applying a compound (Ib) wherein $R^3$ is a benzyl group to debenzylation reaction.

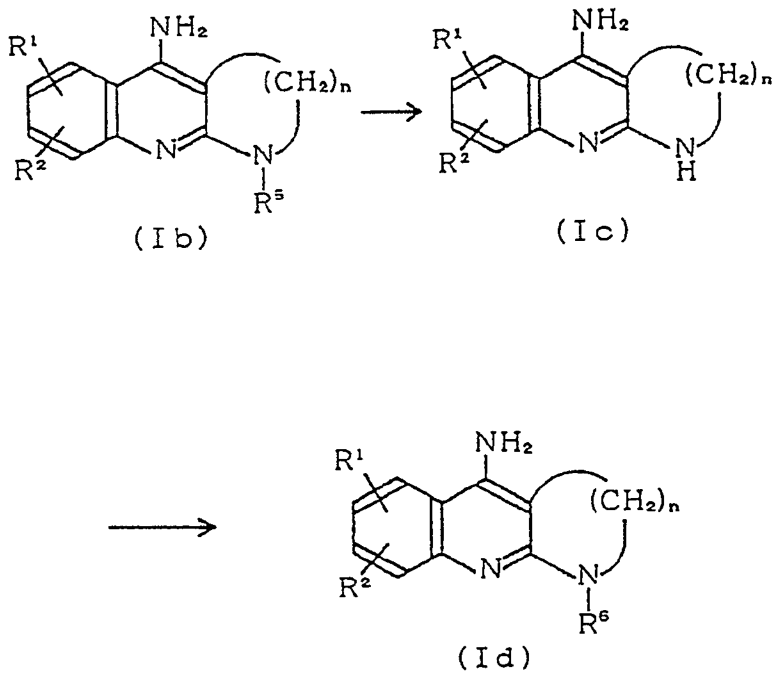

(Ib) (Ic) (Id)

wherein $R^5$ represents a benzyl group, $R^6$ represents an alkanoyl group or a benzoyl group, and $R^1$ and $R^2$ have the same meanings as defined above.

The said reaction can be carried out by optionally selecting a known method such as a reductive cleavage reaction using a palladium catalyst or a debenzylation reaction using boron tribromide or brom cyanide.

Further, in the formula (I), a compound (Id) wherein $R^3$ is an alkanoyl group or a benzoyl group can be prepared by subjecting a compound represented by the formula (Ic) to acylation using a corresponding acid chloride or acid anhydride.

Also, the compound of the formula (Ic) is subjected to alkylation reaction by using an appropriate alkylating

agent such as alkyl halide, aralkyl halide or a sulfonate of an alcohol derivative corresponding to the above halides (e.g. methanesulfonate or paratoluenesulfonate) to produce the compound of the formula (Ib).

The compound of the formula (I) thus prepared can be isolated and purified by optionally employing the conventionally known separating method such as the solvent extraction method, the recrystallization method and the column chromatography method.

On the other hand, the amidine derivative (II) which is a starting material for preparing the compound of the formula (I) can be prepared by condensing a 2-cyanoaniline derivative (III) and a lactam derivative (IV) as shown below.

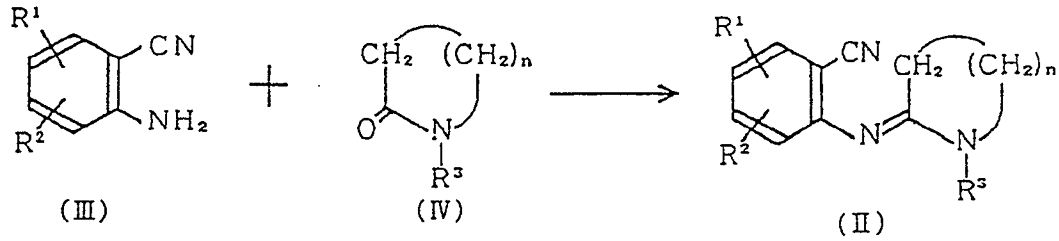

wherein $R^1$, $R^2$, $R^3$ and n have the same meanings as defined above.

The above reaction can be carried out in the presence of an appropriate condensing agent such as phosphorus oxychloride, phosphorus pentchloride and thionyl chloride.

An amount of these condensing agent is generally 0.5 to 10-fold moles, preferably 1.0 to 3-fold moles per mole of the starting material.

As the reaction solvent, it is not particularly limited so long as inactive to the reaction, but preferably halogenated hydrocarbons such as methylene chloride, chloroform and ethylene dichloride ; ethers such as ethyl ether and tetrahydrofuran ; and aromatic hydrocarbons such as benzene and toluene.

A reaction temperature is generally 0°C to 150°C and the reaction is usually carried out at a temperature not more than the boiling point of the reaction solvent.

The compounds of the formula (I) or an acid addition salt thereof inhibit acetylcholine esterase by acting to central neurons of mammals such as human, dog, bovine, rat and mouse, and show beneficial effects on learning and memory task in various amnesia-model animals. Also, since these compounds have low toxicity, they are available as an acetylcholine esterase inhibiting agent, for example, for a prophylaxis and treatment agent of senile dementia and Alzheimer's disease.

When the compounds of the formula (I) and a pharmaceutically acceptable acid addition salt are used as a medicine, it may be administered to a patient required to be treated orally or parenterally as they were or in the form of a medical composition such as powder, grannules, tablets (including film coated tablet and sugar-coated tablet), capsules, injections, suppositories, ointments and poultices by mixing with a pharmaceutically acceptable carrier, excipient or diluent.

A dose may be varied depending on patient's condition, age, weight and method of administration, but in the case of oral administration, it is generally 0.1 to 1000 mg per day, preferably 1 to 500 mg per day and such dose may be administered at once or by dividing into several times.

## EXAMPLES

In the following, the present invention is described by referring to Reference examples and Examples, but the present invention is not limited thereto.

### Reference example 1

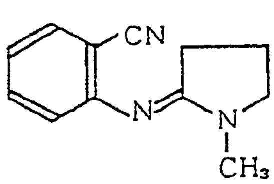

To 20 ml of chloroform solution containing 5.16 g of N-methyl-2-pyrrolidone was added 5.3 ml of phosphorus oxychloride and the mixture was stirred at room temperature for 30 minutes. Subsequently, after

5.85 g of 2-cyanoaniline was added thereto, the mixture was refluxed for 3 hours under heating. After the reaction, about 10 ml of ice water was added thereto and then a 20% aqueous sodium hydroxide solution was further added to make alkaline, and the mixture was extracted with chloroform. The extract was dried over anhydrous sodium sulfate and condensed under reduced pressure to obtain 9.65 g of 2-[(1-methyl-2-pyrrolidinyliden)amino]benzonitrile.

m.p. 84 to 86°C
Mass 199 ($M^+$)
NMR ($CDCl_3$, δ ppm)
1.85-2.08 (m, 2H), 2.31-2.46 (t, 2H), 3.01 (s, 3H), 3.36-3.48 (t, 2H), 6.83-7.02 (m, 2H), 7.33-7.54 (m, 2H)

Reference example 2

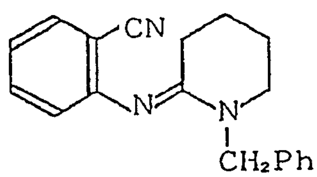

To 100 ml of methylene chloride solution containing 9.46 g of N-benzyl-2-piperidone was added 5.13 ml of phosphorus oxychloride and the mixture was stirred at room temperature for 30 minutes.

Subsequently, after 5.61 g of 2-cyanoaniline was added thereto, the mixture was refluxed for 2.5 hours under heating. After the reaction, about 20 ml of ice water was added thereto and then a 20% aqueous sodium hydroxide solution was further added to make alkaline, and the mixture was extracted with chloroform. The extract was dried over anhydious sodium sulfate and condensed under reduced pressure to obtain 13,7 g of 2-[(1-benzyl-2-piperidinyliden)amino]benzonitrile as pale yellow liquid.

Mass 289 ($M^+$)
NMR ($CDCl_3$, δ ppm)
1.63-1.90 (m, 4H), 2.37 (t, 2H), 3.24 (t, 2H), 4.83 (s, 2H), 6.80-6.90 (dd, 1H), 6.90-7.01 (t, d, 1H), 7.10-7.47 (m, 6H), 7.47-7.58 (dd, 1H)

Reference example 3

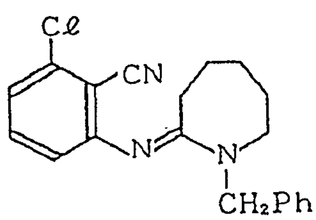

To 75 ml of chloroform solution containing 25.0 g of N-benzylcaprolactam was added 13.0 ml of phosphorus oxychloride and the mixture was stirred at room temperature for 40 minutes. Then, 17.8 g of 3-chloro-2-cyanoaniline was added to the mixture, and the mixture was refluxed for 8 hours under heating.

After the reaction, ice water was added thereto and then a 20% aqueous sodium hydroxide solution was further added to make alkaline, and the mixture was extracted with chloroform. The extract was dried over anhydrous sodium sulfate and condensed under reduced pressure to obtain 32.0 g of pure product of 1-benzyl-2-[(3-chloro-2-cyanophenyl)imino]-hexahydro-1H-azepine.

Mass 337 ($M^+$)
NMR ($CDCl_3$, δ ppm)
1.46-1.75 (m, 6H), 2.40-2.54 (m, 2H), 3.33-3.45 (m, 2H), 4.82 (s, 2H), 6.62-6.70 (d, 1H), 6.96-7.02 (d, 1H), 7.13-7.45 (m, 6H)

Similarly, the following compounds can be obtained.

Reference example 4

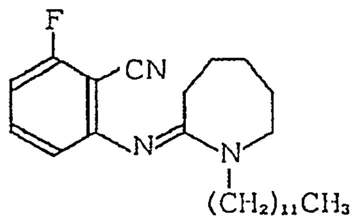

2-(2-Cyano-3-fluorophenylimino)-N-dodecyl-2,3,4,5,6,7-hexahydro-1H-azepine

Oily product
Mass 399 ($M^+$)
NMR ($CDCl_3$, δ ppm),
0.84-0.91 (m, 3H), 1.24-1.31 (m, 18H), 1.50-1.72 (m, 8H), 2.34-2.40 (m, 2H), 3.38-3.46 (m, 2H), 3.47-3.56 (m, 2H), 6.44-6.49 (m, 1H), 6.59-6.68 (m, 1H), 7.24-7.36 (m, 1H)

Reference example 5

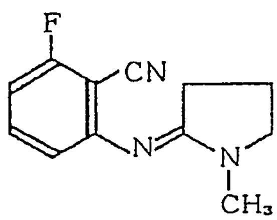

6-Fluoro-(2-[(1-methyl-2-pyrrolidinyliden)amino]benzonitrile

m.p. 95 to 97°C
Mass 217 ($M^+$)
NMR ($CDCl_3$, δ ppm)
1.94-2.12 (m, 2H), 2.38-2.50 (t, 2H), 3.01 (s, 3H), 3.40-3.50 (t, 2H), 6.64-6.75 (m, 2H), 7.28-7.42 (m, 1H)

Reference example 6

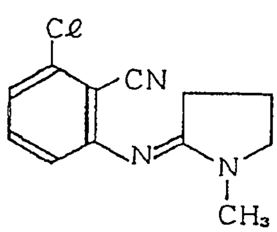

6-Chloro-(2-[(1-methyl-2-pyrrolidinyliden)amino]benzonitrile

m.p. 106 to 108°C
Mass 233 ($M^+$)
NMR ($CDCl_3$, δ ppm)
1.94-2.11 (m, 2H), 2.36-2.48 (t, 2H), 3.03 (s, 3H), 3.40-3.50 (t, 2H), 6.72-6.82 (dd, 1H), 6.98-7.05 (dd, 1H), 7.24-7.35 (m, 1H)

Reference example 7

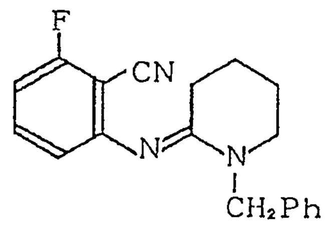

6-Fluoro-2-[(1-benzyl-2-piperidinyliden)amino]benzonitrile

Oily product

Mass 307 ($M^+$)

NMR ($CDCl_3$, δ ppm)

1.63-1.88 (m, 4H), 2.32-2.44 (m, 2H), 3.15-3.33 (m, 2H), 4.80 (s, 2H), 6.55-6.76 (m, 2H), 7.14-7.46 (m, 6H)

Reference example 8

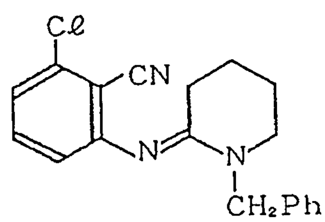

6-Chloro-2-[(1-benzyl-2-piperidinyliden)amino]benzonitrile

Oily product

Mass 323 ($M^+$)

NMR ($CDCl_3$, δ ppm)

1.64-1.89 (m, 4H), 2.34 (t, 2H), 3.25 (t, 2H), 4.80 (s, 2H), 6.66-6.75 (dd, 1H), 6.94-7.03 (dd, 1H), 7.13-7.43 (m, 6H)

Reference example 9

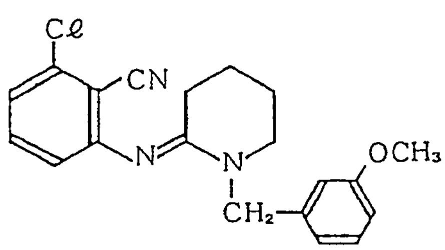

6-Chloro-2-[(1-m-methoxybenzyl-2-piperidinyliden)amino]benzonitrile

Oily product

Mass 353 ($M^+$)

NMR ($CDCl_3$, δ ppm)

1.70-1.88 (m, 4H), 2.36-2.45 (t, 2H), 3.26-3.36 (t, 2H), 3.70 (s, 3H), 4.96 (s, 2H), 6.75-6.86 (m, 2H), 6.92-7.26 (m, 4H), 7.30-7.43 (m, 1H)

Reference example 10

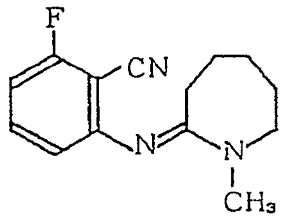

1-Methyl-2-[(2-cyano-3-fluorophenyl)imino]-hexahydro-1H-azepine

m.p. 74 to 75°C
Mass 245 ($M^+$)
NMR ($CDCl_3$, δ ppm)
1.55-1.76 (m, 6H), 2.33-2.45 (m, 2H), 3.12 (s, 3H), 3.40-3.50 (m, 2H), 6.45-6.55 (m, 1H), 6.62-6.73 (m, 1H), 7.28-7.40 (m, 1H)

Reference example 11

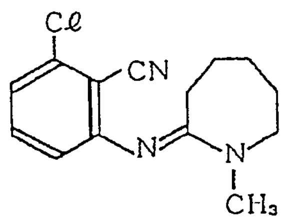

1-Methyl-2-[(2-cyano-3-chlorophenyl)imino]-hexahydro-1H-azepine

m.p. 195 to 199°C
Mass 261 ($M^+$)
NMR ($CDCl_3$, δ ppm)
1.66-1.95 (m, 6H), 2.55-2.78 (m, 2H), 3.36 (s, 3H), 3.79-3.92 (m, 2H), 7.57-7.78 (m, 3H)

Reference example 12

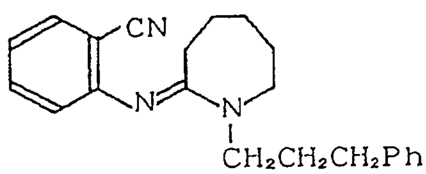

1-(3-Phenylpropyl)-2-[(2-cyanophenyl)imino]hexahydro-1H-azepine

Oily product
Mass 331 ($M^+$)
NMR ($CDCl_3$, δ ppm)
1.50-1.75 (m, 6H), 1.92-2.10 (m, 2H), 2.28-2.42 (m, 2H), 2.65-2.74 (m, 2H), 3.30-3.45 (m, 2H), 3.54-3.67 (m, 2H), 6.67-6.73 (dd, 1H), 6.84-6.96 (m, 1H), 7.10-7.43 (m, 6H), 7.43-7.52 (dd, 1H)

Reference example 13

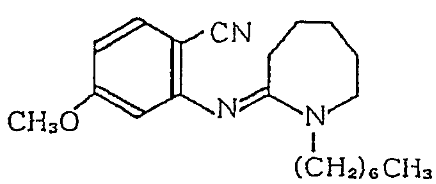

1-Heptyl-2-[(2-cyano-5-methoxyphenyl)imino]hexahydro-1H-azepine

Paly yellow crystals
Mass 341 ($M^+$)
NMR ($CDCl_3$-$DMSOd_6$, $\delta$ ppm)
0.80-0.94 (m, 3H), 1.14-1.42 (m, 8H), 1.55-2.12 (m, 8H), 2.80-3.10 (m, 4H), 3.12-3.28 (m, 2H), 3.97 (s, 3H), 7.09-7.18 (m, 1H), 7.62-7.64 (m, 1H), 8.06-8.12 (m, 1H)

Reference example 14

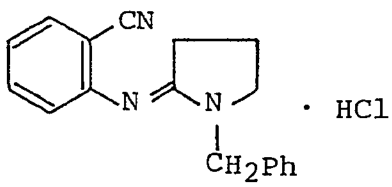

2-[(1-Benzyl-2-pyrrolidinyliden)amino]benzonitrile·hydrochloride

m.p. 167 to 170°C
Mass 275 ($M^+$)
NMR ($CDCl_3$, $\delta$ ppm)
2.07-2.26 (m, 2H), 2.78-2.95 (t, 2H), 3.63-3.77 (t, 2H), 5.44 (s, 2H), 7.30-7.58 (m, 6H), 7.63-7.79 (m, 2H), 7.79-7.90 (m, 1H)

Reference example 15

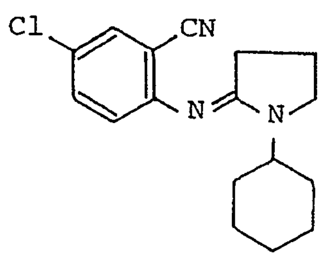

2-[(1-Cyclohexyl-2-pyrrolidinyliden)amino]-5-chlorobenzonitrile

Oily product
Mass 255 ($M^+$)
NMR ($CDCl_3$, $\delta$ ppm)
0.98-1.22 (m, 1H), 1.22-1.56 (m, 4H), 1.60-2.05 (m, 7H), 2.33-2.44 (m, 2H), 3.35-3.42 (t, 2H), 4.00-4.18 (m, 1H), 6.79-6.85 (d, 1H), 7.14-7.26 (m, 1H), 7.27-7.35 (dd, 1H), 7.44-7.46 (d, 1H)

Reference example 16

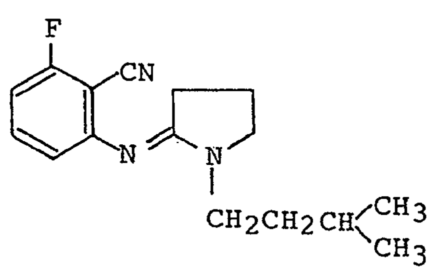

2-[(1-Isoamyl-2-pyrrolidinyliden)amino]-6-fluorobenzonitrile

Oily product

Mass 273 ($M^+$)
MR ($CDCl_3$, δ ppm)
0.92-1.04 (d, 6H), 1.58-1.83 (m, 3H), 2.10-2.25 (m, 2H), 2.71 (t, 2H), 3.73 (t, 2H), 3.91 (t, 2H), 7.00-7.09 (m, 1H), 7.24-7.31 (m, 1H), 7.51-7.63 (m, 1H)

Reference example 17

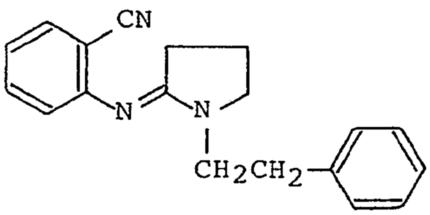

2-[(1-Phenylethyl-2-pyrrolidinyliden)amino]benzonitrile

Oily product
Mass 289 ($M^+$)
NMR ($CDCl_3$, δ ppm)
1.81-1.96 (m, 2H), 2.33-2.41 (t, 2H), 2.98-3.06 (t, 2H), 3.19-3.26 (t, 2H), 3.66-3.73 (t, 2H), 6.82-6.86 (d, 1H), 6.93-7.00 (m, 1H), 7.18-7.31 (m, 5H), 7.35-7.44 (m, 1H), 7.50-7.54 (m, 1H)

Reference example 18

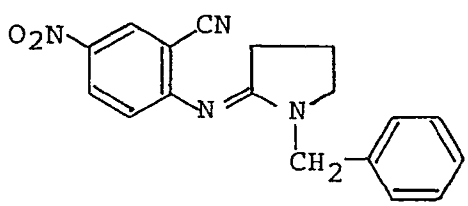

2-[(1-Benzyl-2-pyrrolidinyliden)amino]-5-nitrobenzonitrile

Yellow crystal
m.p. 149 to 151°C
Mass 320 ($M^+$)
NMR ($CDCl_3$-DMSO-$d_6$, δ ppm)
2.08-2.25 (m, 2H), 2.79-2.86 (t, 2H), 3.59-3.66 (t, 2H), 5.05 (s, 2H), 7.34-7.50 (m, 5H), 7.62-7.66 (d, 1H), 8.38-8.46 (m, 1H), 8.52-8.56 (m, 1H)

Reference example 19

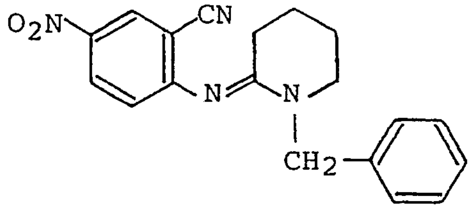

2-[(1-Benzyl-2-piperidinyliden)amino]benzonitrile

Oily product
Mass 334 ($M^+$)
NMR ($CDCl_3$, δ ppm)
1.70-1.90 (m, 4H), 2.38-2.46 (t, 2H), 3.27-3.34 (t, 2H), 4.80 (s, 2H), 6.82-6.86 (d, 1H), 7.20-7.38 (m, 5H),

8.19-8.23 (dd, 1H), 8.39-8.43 (d, 1H)

Example 1

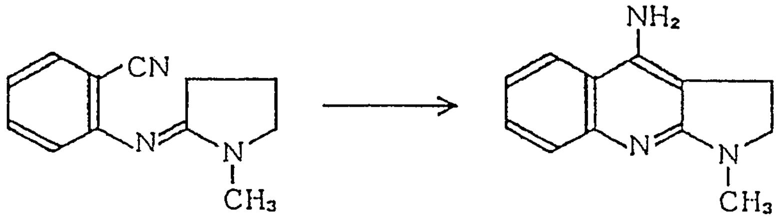

To 30 ml of nitrobenzene solution containing 8.50 g of 2-[(1-methyl-2-pyrrolidinyliden)amino]benzonitrile obtained in Reference example 1 was added 8.1 g of zinc chloride and the mixture was heated to 140°C for 50 minutes. After cooling, the mixture was made alkaline by addition of a 20% aqueous sodium hydroxide solution and then extracted with chloroform. The extract was dried over anhydrous sodium sulfate and condensed under reduced pressure. The residue obtained was applied to column chromatography using silica gel and further recrystallized from ethyl acetate/hexane to obtain 4.32 g of 4-amino-1-methyl-2,3-dihydro-1H-pyrrolo[2,3-b] quinoline.

m.p. 213 to 216°C
Mass 199 ($M^+$)
NMR ($CDCl_3$, δ ppm)
2.87-2.94 (t, 2H), 3.06 (s, 3H), 3.52-3.62 (t, 2H), 4.18 (s, br, 2H), 7.09-7.16 (m, 1H), 7.38-7.51 (m, 2H), 7.62-7.66 (dd, 1H)

Example 2

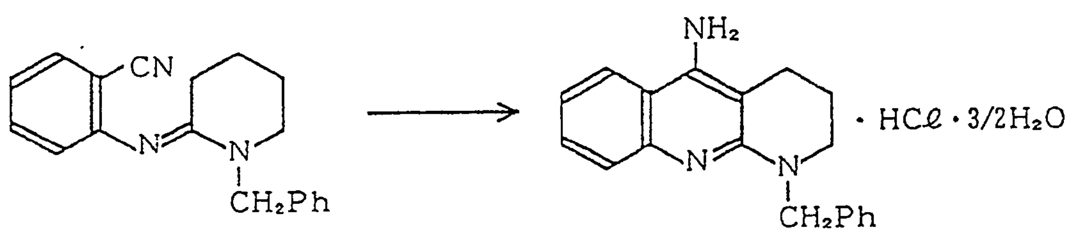

In 80 ml of nitrobenzene was dissolved 24.1 g of 2-[(1benzyl-2-piperidinyliden)amino]benzonitrile obtained in Reference example 2 and after addition of 13.6 g of zinc chloride, the mixture was stirred at 155 to 160°C for 30 minutes. After cooling, the mixture was made alkaline by addition of a 20% aqueous sodium hydroxide solution and then extracted with chloroform. The extract was dried over anhydrous sodium sulfate and condensed under reduced pressure. The residue obtained was applied to column chromatography using silica gel to obtain 18.6 g of 5-amino-1-benzyl-1,2,3,4-tetrahydrobenzo[b] [1,8]naphthylidine as a pale yellow liquid. This liquid was made hydrochloride by adding conc. hydrochloric acid and further recrystallized from ethanol to obtain hydrochloride 3/2 hydrate.

m.p. 237 to 245°C (decomposed)
Mass 289 ($M^+$)
NMR ($CDCl_3$-DMSO-$d_6$, δ ppm)
1.93-2.13 (m, 2H), 2.60-2.72 (t, 2H), 3.45-3.55 (t, 2H), 3.59 (s, br, 2H), 5.09 (s, 2H), 7.24-7.46 (m, 6H), 7.53-7.65 (m, 1H), 7.92-8.01 (d, 1H), 8.22-8.30 (d, 1H)

Example 3

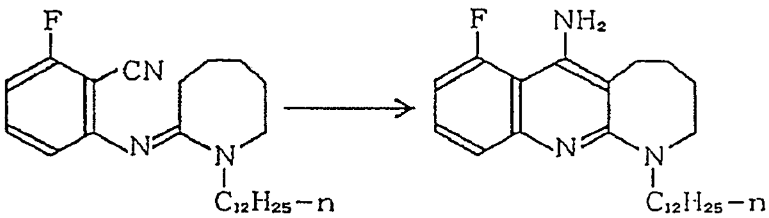

To a nitrobenzene solution containing 11.6 g of 2-(2-cyano-3-fluorophenylimino)-N-dodecyl-2,3,4,5,6,7-hexahydro-1H-azepine obtained in Reference example 4 was added 4.75 g of zinc chloride and the mixture was heated to 140°C for 3.5 hours. After cooling, the mixture was made alkaline of a pH of 9 to 10 by addition of an aqueous ammonia and then extracted with chloroform. The extract was dried over anhydrous sodium sulfate and condensed under reduced pressure. The residue obtained was applied to silica gel column chromatography and further recrystallized from ethanol to obtain 1.06 g of 6-amino-1-dodecyl-7-fluoro-2,3,4,5-tetrahydro-1H-azepino[2,3-b]quinoline.

m.p. 53 to 55°C
Mass 399 ($M^+$)
NM R ($CDCl_3$, δ ppm)
0.84-0.90 (t, 3H), 1.22-1.40 (m, 18H), 1.62-1.66 (m, 2H), 1.78-1.95 (m, 4H), 2.60-2.68 (m, 2H), 3.44-3.50 (t, 2H), 3.58-3.65 (t, 2H), 4.96-5.02 (2H), 6.64-6.76 (dd, 1H), 7.20-7.28 (m, 1H), 7.30-7.38 (m, 1H)

Similarly, the following compounds are obtained.

Example 4

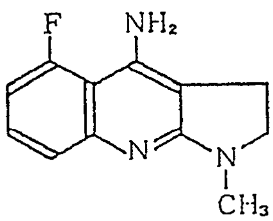

4-Amino-5-fluoro-1-methyl-2,3-dihydro-1H-pyrrolo[2,3-b]-quinoline

m.p. 220 to 222°C
Mass 217 ($M^+$)
NMR ($CDCl_3$, δ ppm)
2.82-2.91 (t, 2H), 3.03 (s, 3H), 3.55-3.62 (t, 2H), 6.66-6.78 (m, 1H), 7.20-7.31 (m, 1H), 7.33-7.41 (dd, 1H)

Example 5

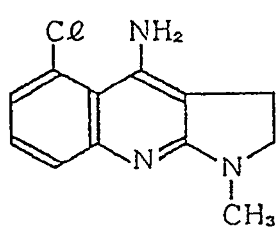

4-Amino-5-chloro-1-methyl-2,3-dihydro-1H-pyrrolo[2,3-b]-quinoline

m.p. 242 to 243°C
Mass 233 ($M^+$)
NMR ($CDCl_3$, δ ppm)

2.81-2.89 (t, 2H), 3.02 (s, 3H), 3.56-3.64 (t, 2H), 7.02-7.06 (dd, 1H), 7.18-7.22 (dd, 1H), 7.49-7.55 (dd, 1H)

Example 6

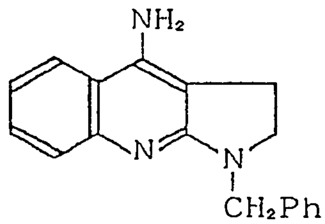

4-Amino-1-benzyl-2,3-dihydro-1H-pyrrolo[2,3-b]quinoline

m.p. 173 to 175°C
Mass 275 ($M^+$)
NMR ($CDCl_3$, δ ppm)
2.88-2.96 (t, 2H), 3.45-3.54 (t, 2H), 4.70 (s, 2H), 7.07-7.15 (t, 1H), 7.20-7.42 (m, 7H), 7.52-7.58 (d, 1H), 7.71 - 7.79 (m, 2H)

The above title compound was prepared by the other preparation method as shown below.

A tetrahydrofuran (650 ml) solution containing 63.2 g of 2-[(1-benzyl-2-pyrrolidinyliden)amino]benzonitrile was cooled to -20°C under argon atmosphere, and then 184 ml of 1.5 mole hexane solution containing lithium diisopropylamide·tetrahydrofuran complex was added thereto dropwise. After dropwise addition, the temperature of the mixture was gradually raised and at -5°C, 50 ml of water was gradually added dropwise. Subsequently, the reaction mixture was extracted with toluene and the toluene solution obtained was washed with a saturated saline solution and dried over anhydrous sodium sulfate. After condensing under reduced pressure, when the residue was treated by a small amount of ethanol, crystals were precipitated. This crystall was collected by filtration to obtain 33.0 g of 4-amino-1-benzyl-2,3-dihydro-1H-pyrrolo[2,3-b]quinoline.

Example 7

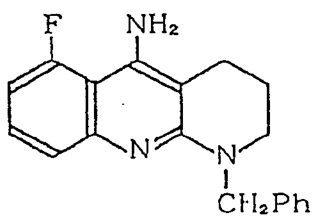

5-Amino-1-benzyl-6-fluoro-1,2,3,4-tetrahydrobenzo[b][1,8]-naphthylidine

m.p. 115 to 117°C
Mass 307 ($M^+$)
NMR ($CDCl_3$, δ ppm)
1.92-2.06 (m, 2H), 2.48-2.59 (m, 2H), 3.23-3.34 (m, 2H), 4.85-5.10 (s, br, 2H), 5.05 (s, 2H), 6.61-6.76 (m, 1H), 7.13-7.38 (m, 7H)

Example 8

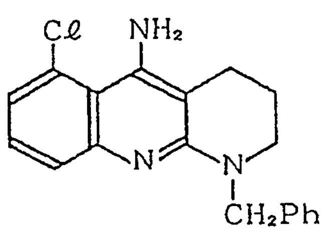

5-Amino-1-benzyl-6-chloro-1,2,3,4-tetrahydrobenzo[b][1,8]-naphthylidine

m.p. 102 to 103°C
Mass 323 ($M^+$)
NMR ($CDCl_3$, δ ppm)
1.90-2.08 (m, 2H), 2.45-2.59 (m, 2H), 3.23-3.34 (m, 2H), 5.04 (s, 2H), 5.48 (s, br, 2H), 6.99-7.08 (dd, 1H), 7.16-7.38 (m, 6H), 7.43-7.52 (dd, 1H)

Example 9

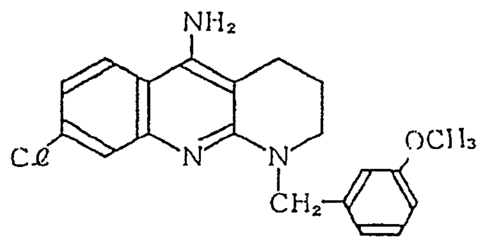

5-Amino-8-chloro-1-(m-methoxybenzyl)-1,2,3,4-tetrahydrobenzo[b][1,8]naphthylidine

m.p. 258 to 260°C
Mass 353 ($M^+$)
NMR ($CDCl_3$-$DMSOd_6$, δ ppm)
1.92-2.10 (m, 2H), 2.66 (t, 2H), 3.45-3.56 (m, 2H), 3.78 (s, 3H), 5.09 (s, 2H), 6.81-6.86 (m, 3H), 7.24-7.32 (m, 2H), 7.46 (s, br, 2H), 8.18-8.19 (d, 1H), 8.25-8.30 (d, 1H)

Example 10

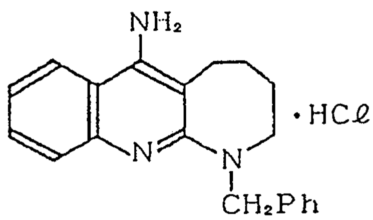

6-Amino-1-benzyl-2,3,4,5-tetrahydro-1H-azepino[2,3-b]-quinoline·hydrochloride

m.p. 260 to 262°C
Mass 303 ($M^+$)
NMR ($CDCl_3$-$DMSOd_6$, δ ppm)
1.70-1.78 (m, 2H), 1.86-1.95 (m, 2H), 2.79-2.86 (m, 2H), 3.54-3.60 (m, 2H), 4.85 (s, 2H), 7.32-7.50 (m, 2H), 8.13-8.18 (d, 1H)

Example 11

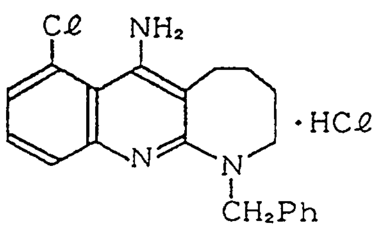

6-Amino-1-benzyl-7-chloro-2,3,4,5-tetrahydro-1H-azepino[2,3-b]quinoline·hydrochloride

m.p. 223 to 236°C
Mass 337 ($M^+$)
NMR ($CDCl_3$, δ ppm)
1.62-1.90 (m, 4H), 2.71-2.85 (m, 2H), 3.15-3.40 (m, 2H), 3.44-3.60 (m, 2H), 5.05 (s, 2H), 7.24-7.54 (m, 7H), 7.62-7.80 (m, 2H), 8.39-8.49 (dd, 1H)

Example 12

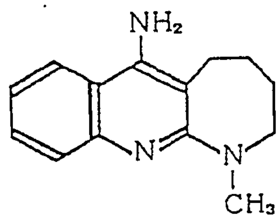

6-Amino-1-methyl-2,3,4,5-tetrahydro-1H-azepino[2,3-b]quinoline

m.p. 259.5 to 261°C
Mass 227 ($M^+$)
NMR ($CDCl_3$, δ ppm)
1.84-2.08 (m, 4H), 2.56-2.72 (m, 2H), 3.32 (s, 3H), 3.53-3.64 (m, 2H), 7.32-7.50 (m, 3H), 7.55-7.66 (m, 1H), 7.98-8.05 (d, 1H), 8.16-8.25 (d, 1H)

Example 13

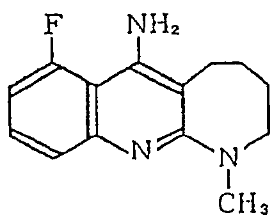

6-Amino-7-fluoro-1-methyl-2,3,4,5-tetrahydro-1H-azepino[2,3-b]quinoline

m.p. 118 to 120°C
Mass 245 ($M^+$)
NMR ($CDCl_3$, δ ppm)
1.81-1.93 (m, 4H), 2.62-2.70 (t, 2H), 3.13 (s, 3H), 3.31-3.40 (t, 2H), 6.68-6.80 (dd, 1H), 7.21-7.33 (m, 1H), 7.36-7.41 (m, 1H)

Example 14

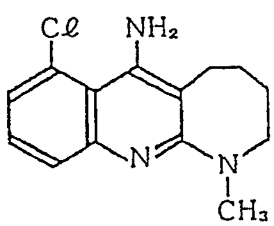

6-Amino-7-chloro-1-methyl-2,3,4,5-tetrahydro-1H-azepino[2,3-b]quinoline

m.p. 95 to 96°C
Mass 261 ($M^+$)
NMR ($CDCl_3$, δ ppm)
1.78-1.96 (m, 4H), 2.58-2.68 (t, 2H), 3.12 (s, 3H), 3.31-3.38 (t, 2H), 7.04-7.09 (dd, 1H), 7.20-7.28 (m,

1H), 7.49-7.55 (dd, 1H)

Example 15

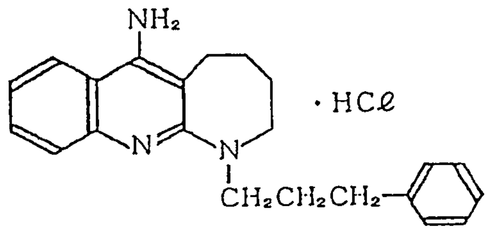

6-Amino-1-(3-phenylpropyl)-2,3,4,5-tetrahydro-1H-azepino[2,3-b]quinoline·hydrochloride

m.p. 198 to 200°C
Mass 331 ($M^+$)
NMR ($CDCl_3$, δ ppm)
1.70-1.90 (m, 2H), 1.90-2.15 (m, 2H), 2.53-2.68 (m, 2H), 2.68-2.85 (m, 2H), 3.30-3.50 (m, 2H), 3.90-4.08 (m, 2H), 6.88 (s, br, 2H), 7.06-7.30 (m, 6H), 7.30-7.40 (m, 1H), 8.10-8.20 (d, 1H), 8.40-8.50 (d, 1H)

Example 16

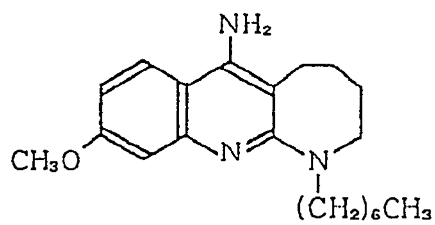

6-Amino-1-heptyl-9-methoxy-2,3,4,5-tetrahydro-1H-azepino[2,3-b]quinoline

m.p. 113 to 114°C
Mass 341 ($M^+$)
NMR ($CDCl_3$-$DMSOd_6$, δ ppm)
0.82-0.90 (t, 3H), 1.20-1.38 (m, 8H), 1.53-2.10 (m, 8H), 2.91-3.20 (m, 4H), 4.00 (s, 3H), 7.12-7.18 (dd, 1H), 7.50 (m, 1H), 8.06-8.13 (d, 1H)

Example 17

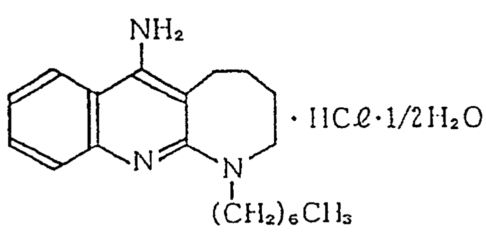

6-Amino-1-heptyl-2,3,4,5-tetrahydro-1H-azepino[2,3-b]quinoline hydrochloride·1/2 hydrate

m.p. 158 to 160°C
Mass 311 ($M^+$)
NMR ($CDCl_3$-$DMSOd_6$, δ ppm)
0.84 (t, 3H), 1.13-1.50 (m, 8H), 1.62-1.81 (m, 2H), 1.82-2.01 (m, 4H), 2.80-2.98 (m, 2H), 3.46-3.62 (t, 2H), 3.90 (t, 2H), 7.10 (s, 2H), 7.30 (m, 1H), 7.52 (m, 1H), 8.13-8.20 (m, 1H), 8.46-8.50 (m, 1H)

Example 18

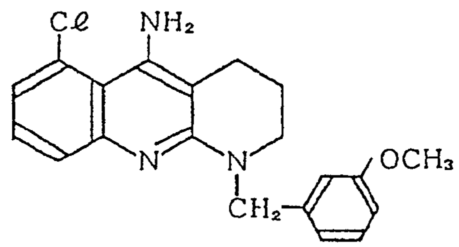

5-Amino-6-chloro-1-(m-methoxybenzyl)-1,2,3,4-tetrahydrobenzo[b][1,8]naphthylidine

m.p. 97 to 98.5°C
Mass 353 ($M^+$)
NMR ($CDCl_3$, δ ppm)
1.90-2.02 (m, 2H), 2.43-2.50 (m, 2H), 3.23-3.29 (m, 2H), 3.72 (s, 3H), 4.99 (s, 2H), 5.44 (s, br, 2H), 6.72-6.78 (m, 1H), 6.87-6.91 (m, 2H), 6.99-7.03 (m, 1H), 7.14-7.23 (m, 2H), 7.43-7.47 (m, 1H)

Example 19

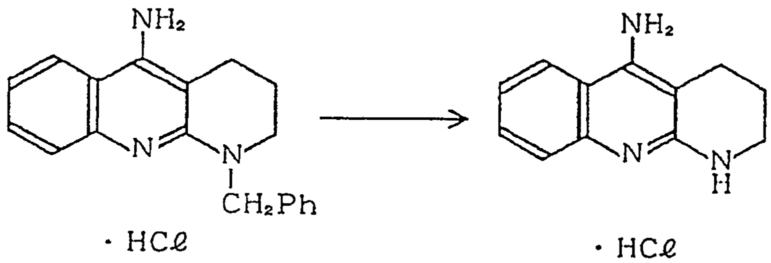

In a mixed solvent of 50 ml of ethanol and 30 ml of acetic acid was dissolved 3.29 g of 5-amino-1-benzyl-1,2,3,4-tetrahydrobenzo[b] [1,8]naphthylidine·hydrochloride·2/3 hydrate, and 1.0 g of a 5% palladium carbon was added to the mixture and then hydrogen reduction was carried out at 70°C under normal pressure for 5 hours. After the reaction, the catalyst was removed by filtration and the filtrate was condensed to obtain 2.07 g of 5-amino-1,2,3,4-tetrahydrobenzo[b] [1,8]naphthylidine·hydrochloride as white crystals.

m.p. 242 to 247°C (decomposed)
Mass 199 ($M^+$)
NMR ($CDCl_3$-$DMSOd_6$, δ ppm)
1.86-2.10 (m, 2H), 2.55-2.70 (m, 2H), 3.30-3.60 (m, 2H), 7.22-7.35 (m, 1H), 7.35-7.65 (m, 4H), 7.79 (s, br, 1H), 8.19-8.30 (dd, 1H)

Example 20

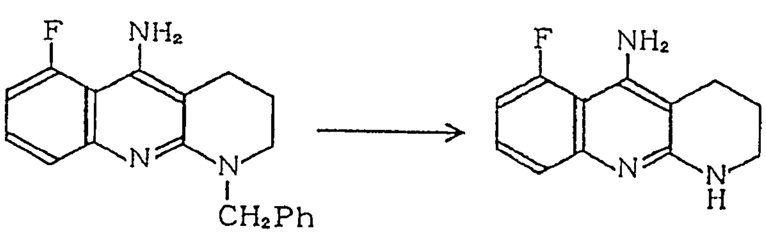

In a mixed solvent of 60 ml of dioxane and 30 ml of acetic acid was dissolved 9.25 g of 5-amino-1-benzyl-6-fluoro-1,2,3,4-tetrahydrobenzo[b] [1,8]naphthylidine, and 1.0 g of a 5% palladium carbon was added to the mixture and then hydrogen reduction was carried out at 75°C under normal pressure for 7 hours. After the reaction, the catalyst was removed by filtration and the filtrate was condensed under reduced pressure. After the residue was neutralized by adding a 20% aqueous sodium hydroxide, the mixture was again condensed under reduced pressure. The residue obtained was applied to column chromatography using silica gel to obtain 4.39 g of 5-amino-6-fluoro-1,2,3,4-tetrahydrobenzo[d] [1,8]naphthylidine.

m.p. 188 to 190°C
Mass 217 ($M^+$) 216 ($M^+$-1)
NMR ($CDCl_3$, δ ppm)
1.93-2.10 (m, 2H), 2.44-2.58 (t, 2H), 3.35-3.47 (t, 2H), 5.62 (s, br, 2H), 6.62-6.83 (m, 1H), 7.20-7.35 (m, 2H)

Example 21

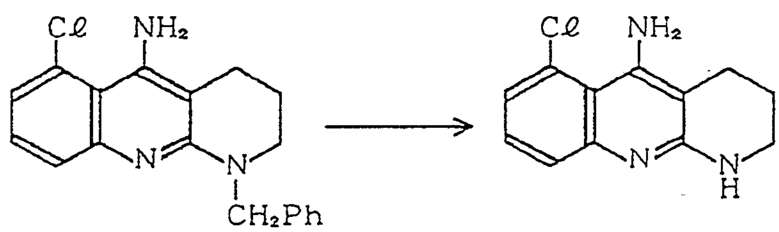

After dissolving 7.33 g of 5-amino-1-benzyl-6-chloro-1,2,3,4-tetrahydrobenzo[b] [1,8]naphthylidine in 50 ml of methylene chloride, 31.4 ml of boron tribromide was added dropwise to the mixture under ice-cooling. After completion of dropwise addition, the mixture was stirred under room temperature for 6 hours. The reaction mixture was then poured into ice-water, then the mixture was made alkaline by adding a 30% aqueous sodium hydroxide solution. The mixture was extracted with chloroform containing a little amount of ethanol and dried over anhydrous sodium sulfate. The solvent was removed under reduced pressure, and the residue obtained was applied to column chromatography using silica gel to obtain 4.23 g of 5-amino-6-chloro-1,2,3,4-tetrahydrobenzo[b] [1,8]naphthylidine.

m.p. 196 to 200°C
Mass 233 ($M^+$) 232 ($M^+$-1)
NMR ($CDCl_3$, δ ppm)
1.93-2.12 (m, 2H), 2.43-2.56 (m, 2H), 3.32-3.45 (m, 2H), 5.40 (s, br, 1H), 5.60 (s, br, 1H), 6.98-7.08 (dd, 1H), 7.13-7.28 (m, 1H), 7.32-7.42 (dd, 1H)

Similarly, the following compounds can be obtained.

Example 22

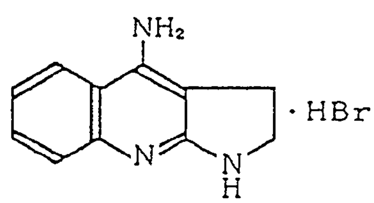

4-Amino-2,3-dihydro-1H-pyrrolo[2,3-b]quinoline·hydrobromide

m.p. >300°C
Mass 185 ($M^+$)
NMR ($CDCl_3$-$DMSOd_6$, δ ppm)
3.03-3.18 (t, 2H), 3.82-3.95 (t, 2H), 7.24 (s, 2H), 7.26-7.38 (m, 1H), 7.52-7.59 (m, 2H), 7.86 (s, br, 1H), 8.15-8.22 (d, 1H)

Example 23

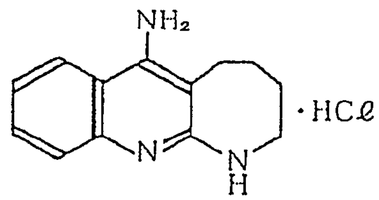

6-Amino-2,3,4,5-tetrahydro-1H-azepino[2,3-b]quinoline--hydrochloride

m.p. 222 to 225°C
Mass 213 ($M^+$)
NMR ($CDCl_3$-$DMSOd_6$, δ ppm)
1.78-2.10 (m, 6H), 3.53-3.64 (m, 2H), 6.65 (s, br, 2H), 7.22-7.32 (m, 1H), 7.50-7.61 (m, 2H), 7.87-8.01 (d, 1H), 13.35 (s, br, 1H)

Example 24

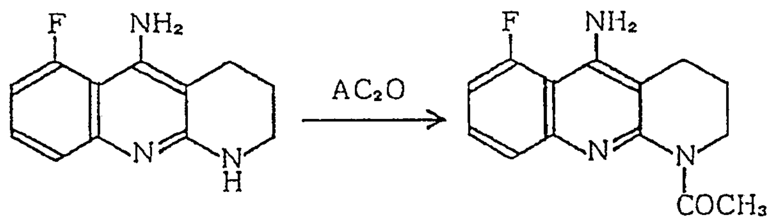


To a mixed solutionof 5 ml of anhydrous acetic acid and 5 ml of acetic acid was added 1.27 g of 5-amino-6-fluoro-1,2,3,4-tetrahydrobenzo[b] [1,8]naphthylidine, and the mixture was stirred at 50°C for 30 minutes. The mixture was condensed under reduced pressure, and the residue obtained was applied to column chromatography using silica gel to obtain 0.65 g of 1-acetyl-5-amino-6-fluoro-1,2,3,4-tetrahydrobenzo[b] [1,8]naphthylidine.

m.p. 149 to 153°C
Mass 259 ($M^+$)
NMR ($CDCl_3$, δ ppm)
1.96-2.13 (m, 2H), 2.54-2.65 (m, 2H), 2.58 (s, 3H), 3.93-4.02 (m, 2H), 5.36 (s, br, 2H), 6.90-7.04 (m, 1H), 7.35-7.60 (m, 2H)

Example 25

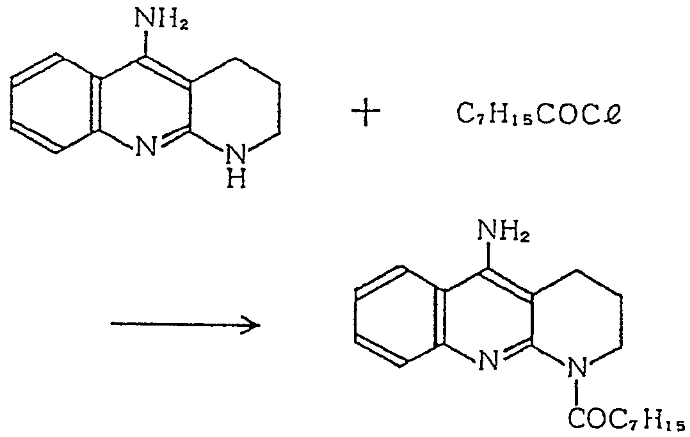


In a mixed solvent of 25 ml of dioxane and 5 ml of dimethylformamide was dissolved 1.00 g of 5-amino-1,2,3,4-tetrahydrobenzo[b] [1,8]naphthylidine, and then 1.40 g of triethylamine and 1.3 ml of octanoyl chloride

were added to the mixture and the mixture was stirred at room temperature for 12 hours. After removing the solvent under reduced pressure, the residue was dissolved in chloroform and washed with a 1N aqueous sodium hydroxide solution. The chloroform layer was dried over anhydrous sodium sulfate, and then condensed under reduced pressure. The residue obtained was applied to column chromatography using silica gel to obtain 5-amino-1-octanoyl-1,2,3,4-tetrahydrobenzo-[b] [1,8]naphthylidine as a colorless liquid. This was converted into hydrochloride by using conc. hydrochloric acid and recrystallized from ethyl acetate-ethanol to obtain 0.44 g of hydrochloride·1/2 hydrate.

m.p. 193 to 199°C (decomposed)
Mass 325 ($M^+$)
NMR ($CDCl_3$, δ ppm)
0.88 (t, 3H), 1.20-1.45 (m, 8H), 1.67-1.80 (m, 2H), 2.10-2.25 (m, 2H), 2.76-2.90 (m, 2H), 2.90-3.00 (m, 2H), 3.95-4.10 (m, 2H), 7.33-7.47 (m, 1H), 7.47-7.55 (m, 1H), 7.63-7.71 (m, 1H), 8.98-9.05 (m, 1H)

Example 26

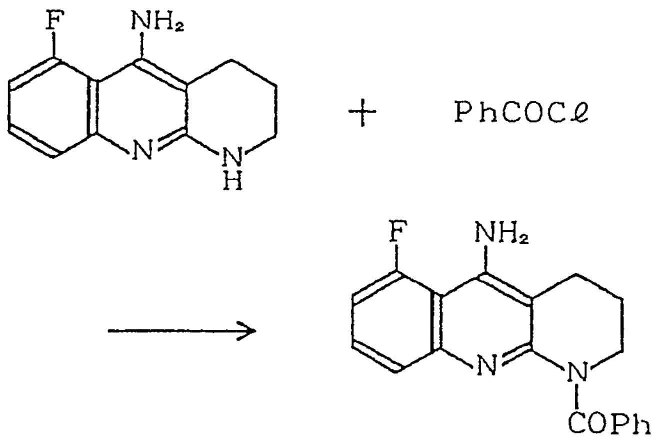

In a mixed solvent of 5 ml of dioxane and 5 ml of dimethylformamide was dissolved 1.44 g of 5-amino-6-fluoro-1,2,3,4-tetrahydrobenzo[b] [1,8]naphthylidine, and then 1.2 g of benzoyl chloride and 1.8 ml of triethylamine were added to the mixture and the mixture was stirred at room temperature for 12 hours. After removing the solvent under reduced pressure, the residue was dissolved in chloroform and washed with a 1N aqueous sodium hydroxide solution. The chloroform layer was dried over anhydrous sodium sulfate, and then condensed under reduced pressure. The residue obtained was applied to column chromatography using silica gel to obtain 0.84 g of 5-amino-1-benzoyl-1,2,3,4-tetrahydrobenzo[b] [1,8]naphthylidine.

m.p. 179 to 183°C (decomposed)
Mass 321 ($M^+$)
NMR ($CDCl_3$, δ ppm)
2.14-2.32 (m, 2H), 2.63-2.76 (m, 2H), 4.00-4.15 (m, 2H), 5.15-5.50 (d, 2H), 6.75-6.93 (m, 2H), 7.08-7.33 (m, 4H), 7.33-7.43 (m, 2H)

In the same manner as mentioned above, the following compounds were prepared.

Example 27

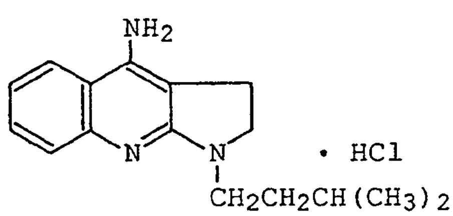

4-Amino-1-isoamyl-2,3-dihydro-1H-pyrrolo[2,3-b]quinoline-hydrochloride

m.p. 256 to 258°C
Mass 255 ($M^+$)
NMR ($CDCl_3$, δ ppm)
0.97-1.00 (d, 6H), 1.50-1.64 (m, 2H), 1.69-1.89 (m, 1H), 3.05-3.12 (t, 2H), 3.75-3.88 (m, 2H)

Example 28

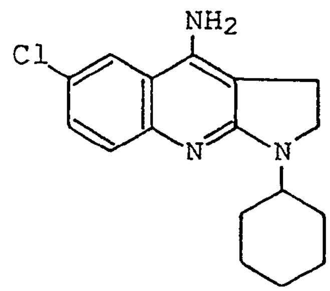

4-Amino-6-chloro-1-cyclohexyl-2,3-dihydro-1H-pyrrolo[2,3-b]quinoline

m.p. 214 to 215°C
Mass 319 ($M^+$)
NMR ($CDCl_3$, δ ppm)
1.00-1.90 (m, 10H), 2.85-2.92 (t, 2H), 3.61-3.69 (t, 1H), 4.00-4.30 (m, 3H), 7.32-7.36 (m, 1H), 7.41-7.43 (m, 1H), 7.53-7.57 (m, 1H)

Example 29

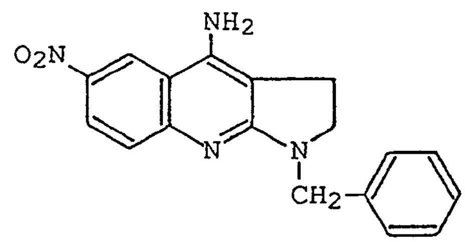

4-mino-6-nitro-1-benzyl-2,3-dihydro-1H-pyrrolo[2,3-b]quinoline

m.p. 147 to 148°C
Mass 320 ($M^+$)
NMR ($CDCl_3$-DMSO-$d_6$, δ ppm)
1.98-2.13 (m, 2H), 2.75-2.88 (m, 2H), 3.85 (s, 2H), 7.23-7.40 (m, 5H), 7.67-7.74 (d, 1H), 8.44-8.52 (m, 1H), 9.04-9.06 (m, 1H)

Example 30

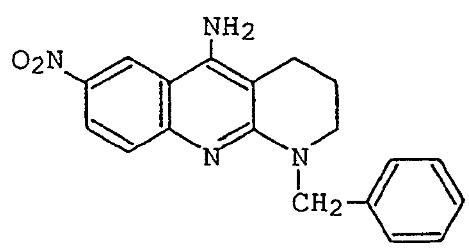

5-Amino-7-nitro-1-benzyl-1,2,3,4-tetrahydrobenzo[b] [1,8]naphthylidine

m.p. 194 to 195°C

Mass 334 ($M^+$)
NMR ($CDCl_3$-DMSO-$d_6$, δ ppm)
1.93-2.06 (m, 2H), 2.50-2.66 (m, 2H), 3.35-3.40 (m, 2H), 5.08 (s, 2H), 7.23-7.33 (m, 5H), 7.43-7.50 (d, 1H), 8.10-8.18 (m, 1H), 8.77-8.80 (m, 1H)

Example 31

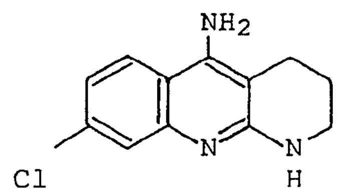

5-Amino-8-chloro-1,2,3,4-tetrahydrobenzo[b] [1,8]naphthylidine

m.p. 220 to 221.5°C
Mass 232 ($M^+$)
NMR ($CDCl_3$, δ ppm)
1.95-2.12 (m, 2H), 2.53-2.65 (t, 2H), 3.38-3.47 (t, 2H), 4.33-4.50 (m, 2H), 7.03-7.06 (m, 1H), 7.39-7.45 (m, 1H), 7.47-7.49 (m, 1H)

Example 32

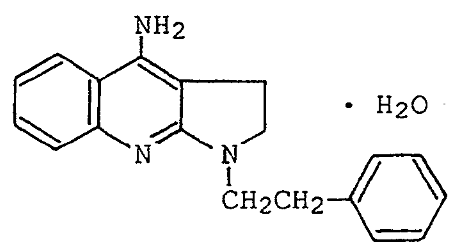

4-Amino-1-phenylethyl-2,3-dihydro-1H-pyrrolo[2,3-b]quinoline·monohydrate

m.p. 144.5 to 151°C
Mass 289 ($M^+$)
NMR ($CDCl_3$, δ ppm)
1.85-1.96 (m, 2H), 2.79-2.92 (m, 4H), 2.96-3.02 (t, 2H), 7.21-7.32 (m, 5H), 7.39-7.47 (m, 1H), 7.61-7.75 (m, 2H), 8.25-8.30 (m, 1H)

Example 33

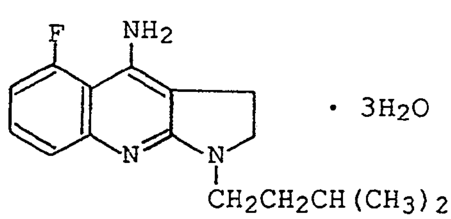

4-Amino-5-fluoro-1-isoamyl-2,3-dihydro-1H-pyrrolo[2,3-b]quinoline·trihydrate

m.p. 217 to 218°C
Mass 213 ($M^+$)
NMR ($CDCl_3$-DMSO-$d_6$, δ ppm)
0.87-1.02 (m, 1H), 0.96-0.98 (d, 6H), 1.45-1.59 (m, 2H), 1.60-1.80 (m, 2H), 2.78-3.00 (m, 4H), 4.85-5.00 (s, 2H), 6.62-6.78 (m, 1H), 7.23-7.32 (m, 1H), 7.68-8.56 (m, 1H)

Example 34

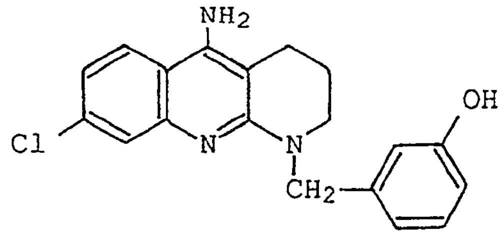

5-Amino-8-chloro-1-(m-hydroxybenzyl)-1,2,3,4-tetrahydrobenzo[b] [1,8]naphthylidine

m.p. 253 to 254°C
Mass 339 ($M^+$)
NMR ($CDCl_3$-DMSO-$d_6$, δ ppm)
1.91-2.06 (m, 2H), 2.55-2.69 (m, 4H), 3.26-3.38 (m, 2H), 4.92 (s, 2H), 5.33 (s, 2H), 6.60-6.74 (m, 1H), 6.75 (s, 2H), 6.92-7.00 (m, 1H), 7.01-7.12 (m, 1H), 7.38-7.41 (m, 1H), 7.70-7.78 (m, 1H)

In the following, preferred compounds of the present invention are enumerated in Table 1.

Table 1

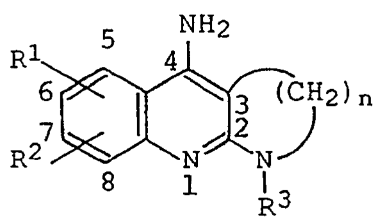

| $R^1$ | $R^2$ | n | $R^3$ | $R^1$ | $R^2$ | n | $R^3$ |
|---|---|---|---|---|---|---|---|
| H | H | 2 | H | H | H | 2 | 4-chlorobenzyl |
| H | H | 2 | methyl | H | H | 2 | 3-chlorobenzyl |
| H | H | 2 | ethyl | H | H | 2 | 4-methoxybenzyl |
| H | H | 2 | n-propyl | H | H | 2 | 3-methoxybenzyl |
| H | H | 2 | i-propyl | H | H | 2 | 4-hydroxybenzyl |
| H | H | 2 | n-butyl | H | H | 2 | 3-hydroxybenzyl |
| H | H | 2 | i-butyl | H | H | 2 | 4-methylbenzyl |
| H | H | 2 | n-pentyl | H | H | 2 | 4-fluorobenzyl |
| H | H | 2 | i-pentyl | H | H | 2 | 2-phenylethyl |
| H | H | 2 | n-hexyl | H | H | 2 | 3-phenylethyl |
| H | H | 2 | n-heptyl | H | H | 2 | acetyl |
| H | H | 2 | n-octyl | H | H | 2 | propanoyl |
| H | H | 2 | n-dodecyl | H | H | 2 | n-butanoyl |
| H | H | 2 | cyclopentyl | H | H | 2 | n-hexanoyl |
| H | H | 2 | cyclohexyl | H | H | 2 | n-octanoyl |
| H | H | 2 | benzyl | H | H | 2 | benzoyl |

Table 1 (Contd)

| $R^1$ | $R^2$ | n | $R^3$ | $R^1$ | $R^2$ | n | $R^3$ |
|---|---|---|---|---|---|---|---|
| H | H | 2 | 4-chlorobenzoyl | H | H | 4 | n-dodecyl |
| H | H | 2 | 3-chlorobenzoyl | H | H | 4 | cyclohexyl |
| H | H | 2 | 4-methoxybenzoyl | H | H | 4 | benzyl |
| H | H | 2 | 3-methoxybenzoyl | H | H | 4 | 4-chlorobenzyl |
| H | H | 2 | 4-hydroxybenzoyl | H | H | 4 | 4-methoxybenzyl |
| H | H | 3 | H | H | H | 4 | 4-hydroxybenzyl |
| H | H | 3 | methyl | H | H | 4 | 2-phenylethyl |
| H | H | 3 | ethyl | H | H | 4 | 3-phenylpropyl |
| H | H | 3 | n-propyl | H | H | 4 | acetyl |
| H | H | 3 | cyclopentyl | H | H | 4 | propanoyl |
| H | H | 3 | cyclohextl | H | H | 4 | n-butanoyl |
| H | H | 3 | benzyl | H | H | 4 | n-octanoyl |
| H | H | 3 | 4-chlorobenzyl | H | H | 4 | benzyl |
| H | H | 3 | 3-chlorobenzyl | H | H | 4 | 4-chlorobenzyl |
| H | H | 3 | 4-methoxybenzyl | H | H | 4 | 4-methoxybenzyl |
| H | H | 3 | 3-methoxybenzyl | 5-F | H | 2 | H |
| H | H | 3 | 4-hydroxybenzyl | 5-Cl | H | 2 | H |
| H | H | 3 | 2-phenylethyl | 6-Cl | H | 2 | H |
| H | H | 3 | 3-phenylpropyl | 7-Cl | H | 2 | H |
| H | H | 3 | acetyl | 7-$OCH_3$ | H | 2 | H |
| H | H | 3 | propanoyl | 7-$CH_3$ | H | 2 | H |
| H | H | 3 | n-butanoyl | 6-$NO_2$ | H | 2 | H |
| H | H | 3 | n-octanoyl | 7-$NO_2$ | H | 2 | H |
| H | H | 3 | benzoyl | 7-OH | H | 2 | H |
| H | H | 3 | 4-chlorobenzoyl | 6-$CF_3$ | H | 2 | H |
| H | H | 3 | 4-methoxybenzoyl | 5-F | H | 3 | H |
| H | H | 3 | 4-methylbenzoyl | 5-Cl | H | 3 | H |
| H | H | 4 | H | 6-Cl | H | 3 | H |
| H | H | 4 | methyl | 7-Cl | H | 3 | H |
| H | H | 4 | ethyl | 7-$OCH_3$ | H | 3 | H |
| H | H | 4 | n-butyl | 6-$NO_2$ | H | 3 | H |
| H | H | 4 | n-heptyl | 5-F | H | 4 | H |

Table 1 (Contd)

| $R^1$ | $R^2$ | n | $R^3$ | $R^1$ | $R^2$ | n | $R^3$ |
|---|---|---|---|---|---|---|---|
| 5-Cl | H | 4 | H | 6-NHAc | H | 2 | benzyl |
| 6-Cl | H | 4 | H | 5-F | H | 3 | benzyl |
| 7-Cl | H | 4 | H | 5-Cl | H | 3 | benzyl |
| 7-$OCH_3$ | H | 4 | H | 6-Cl | H | 3 | benzyl |
| 5-F | H | 2 | methyl | 7-Cl | H | 3 | benzyl |
| 5-Cl | H | 2 | methyl | 6-$OCH_3$ | H | 3 | benzyl |
| 7-Cl | H | 2 | methyl | 7-$OCH_3$ | H | 3 | benzyl |
| 7-$OCH_3$ | H | 2 | methyl | 6-$NO_2$ | H | 3 | benzyl |
| 7-OH | H | 2 | methyl | 5-F | H | 4 | benzyl |
| 7-OAc | H | 2 | methyl | 5-Cl | H | 4 | benzyl |
| 6-$NO_2$ | H | 2 | methyl | 6-Cl | H | 4 | benzyl |
| 6-$NH_2$ | H | 2 | methyl | 7-Cl | H | 4 | benzyl |
| 6-NHAc | H | 2 | methyl | 6-$OCH_3$ | H | 4 | benzyl |
| 5-F | H | 3 | methyl | 5-F | H | 2 | n-butyl |
| 5-Cl | H | 3 | methyl | 5-F | H | 2 | n-pentyl |
| 6-Cl | H | 3 | methyl | 5-F | H | 2 | i-pentyl |
| 7-Cl | H | 3 | methyl | 5-F | H | 2 | n-hexyl |
| 7-$OCH_3$ | H | 3 | methyl | 5-F | H | 2 | cyclohexyl |
| 5-F | H | 4 | methyl | 5-F | H | 2 | 4-chlorobenzyl |
| 5-Cl | H | 4 | methyl | 5-F | H | 2 | 4-methoxybenzyl |
| 6-Cl | H | 4 | methyl | 5-F | H | 2 | acetyl |
| 7-Cl | H | 4 | methyl | 5-F | H | 2 | propanoyl |
| 7-$OCH_3$ | H | 4 | methyl | 5-F | H | 2 | n-butanoyl |
| 5-F | H | 2 | methyl | 5-F | H | 2 | benzoyl |
| 5-Cl | H | 2 | benzyl | 5-F | H | 2 | 4-chlorobenzoyl |
| 6-Cl | H | 2 | benzyl | 5-F | H | 2 | 4-methoxybenzoyl |
| 7-Cl | H | 2 | benzyl | 5-F | H | 3 | 4-chlorobenzyl |
| 6-$OCH_3$ | H | 2 | benzyl | 5-F | H | 3 | 4-methoxybenzyl |
| 7-$OCH_3$ | H | 2 | benzyl | 5-F | H | 3 | 4-hydroxybenyl |
| 6-$NO_2$ | H | 2 | benzyl | 5-F | H | 3 | acetyl |
| 7-$NO_2$ | H | 2 | benzyl | 5-F | H | 3 | propanoyl |
| 6-$NH_2$ | H | 2 | benzyl | 5-F | H | 3 | benzoyl |

Table 1 (Contd)

| $R^1$ | $R^2$ | n | $R^3$ | $R^1$ | $R^2$ | n | $R^3$ |
|---|---|---|---|---|---|---|---|
| 5-F | H | 3 | 4-chlorobenzoyl | 6-$OCH_3$ | H | 2 | benzoyl |
| 5-F | H | 3 | 4-methoxybenzoyl | 7-$OCH_3$ | H | 2 | n-butyl |
| 5-F | H | 4 | octyl | 7-$OCH_3$ | H | 2 | acetyl |
| 5-F | H | 4 | dodecyl | 7-$OCH_3$ | H | 2 | benzoyl |
| 5-F | H | 4 | 4-chlorobenzyl | 7-$OCH_3$ | H | 3 | acetyl |
| 5-F | H | 4 | 4-methoxybenzyl | 7-$OCH_3$ | H | 3 | benzoyl |
| 5-Cl | H | 2 | 4-chlorobenzyl | 7-$OCH_3$ | H | 4 | n-butyl |
| 5-Cl | H | 2 | 4-methoxybenzyl | 7-$OCH_3$ | H | 4 | n-hexyl |
| 5-Cl | H | 2 | acetyl | 7-$OCH_3$ | H | 4 | n-heptyl |
| 5-Cl | H | 2 | benzoyl | 6-$NO_2$ | H | 2 | 4-chlorobenzyl |
| 5-Cl | H | 3 | 4-chlorobenzyl | 6-$NO_2$ | H | 2 | 4-methoxybenzyl |
| 5-Cl | H | 3 | 4-methoxybenzyl | 6-$NO_2$ | H | 2 | acetyl |
| 5-Cl | H | 3 | 3-methoxybenzyl | 6-$NO_2$ | H | 2 | benzoyl |
| 5-Cl | H | 3 | acetyl | 6-$NO_2$ | H | 3 | 4-chlorobenzyl |
| 5-Cl | H | 3 | benzoyl | 6-$NO_2$ | H | 3 | 4-methoxybenzyl |
| 7-Cl | H | 2 | 4-methoxybenzyl | 6-$NO_2$ | H | 3 | acetyl |
| 7-Cl | H | 2 | 3-methoxybenzyl | 6-$NO_2$ | H | 3 | benzoyl |
| 7-Cl | H | 2 | acetyl | 7-$NO_2$ | H | 4 | acetyl |
| 7-Cl | H | 2 | benzoyl | 7-$NO_2$ | H | 4 | benzoyl |
| 7-Cl | H | 3 | 4-methoxybenzyl | 5-Cl | 7-Cl | 2 | H |
| 7-Cl | H | 3 | 3-methoxybenzyl | 5-Cl | 7-Cl | 2 | methyl |
| 7-Cl | H | 3 | 4-hydroxybenzyl | 5-Cl | 7-Cl | 2 | benzyl |
| 7-Cl | H | 3 | 3-hydroxybenzyl | 5-Cl | 7-Cl | 3 | H |
| 7-Cl | H | 3 | acetyl | 5-Cl | 7-Cl | 3 | methyl |
| 7-Cl | H | 3 | benzoyl | 5-Cl | 7-Cl | 3 | benzyl |
| 6-Cl | H | 2 | butyl | 6-$OCH_3$ | 7-$OCH_3$ | 2 | H |
| 6-Cl | H | 2 | cyclohexyl | 6-$OCH_3$ | 7-$OCH_3$ | 2 | methyl |
| 6-Cl | H | 2 | acetyl | 6-$OCH_3$ | 7-$OCH_3$ | 2 | benzyl |
| 6-Cl | H | 2 | benzoyl | 6-$OCH_3$ | 7-$OCH_3$ | 3 | H |
| 6-Cl | H | 3 | acetyl | 6-$OCH_3$ | 7-$OCH_3$ | 3 | methyl |
| 6-Cl | H | 3 | benzoyl | 6-$OCH_3$ | 7-$OCH_3$ | 3 | benzyl |
| 6-$OCH_3$ | H | 2 | acetyl | | | | |

Experimental example

In order to show effects of the compounds according to the present invention in more detail, a part of experimental examples relating to action against scopolamine-induced amnesia for step-through passive avoidance response in rats is shown below.

Experimental method

Wistar male rats of 7 to 8 weeks old were used to experiment after fasting for 12 hours. Rats were divided into groups with 8 rats per group and 1.5 mg/kg of scopolamine was intraperitoneally administered. After 30 minutes from the administration, rats were placed in a light compartment of a step-through type passive avoidance apparatus constituted by two compartments (25 × 10 × 25 cm of a light compartment and 30 × 30 × 30 cm of a dark compartment having a guillotine door and a floor grid for passing current) and a latent time by entering into a dark compartment was measured. The guillotine door was shut when a rat entered into the dark compartment and shock with 0.6 mA for 3 seconds was applied through the floor grid for acquisition trial. After acquisition trial, 10 mg/kg of each test compound was orally administered to rats and returned to their home cage. After 24 hours, rats were again placed in the light compartment at which the guillotine door was opened, a latent time by entering into the dark compartment was measured whereby presence or absence of improved effect of memory retention was judged. The results are shown in Table 2.

Results

From Table 2 set forth below, it can be understood that the compounds of the present invention have excellent antiamnestic action.

Table 2

| Compound (Example No.) | Administered dose (mg/kg p.o.) | Latent time of memory retention (sec) |
|---|---|---|
| 1 | 10 | 128 |
| 3 | 10 | 140 |
| 10 | 10 | 188 |
| 11 | 10 | 163 |
| 20 | 10 | 59 |
| 21 | 10 | 117 |
| 22 | 10 | 78 |
| 24 | 10 | 168 |
| 26 | 10 | 84 |
| 31 | 10 | 72 |
| 33 | 10 | 151 |
| Tacrine | 10 | 58 |

## Claims

1. A quinoline compound represented by the following formula (I) :

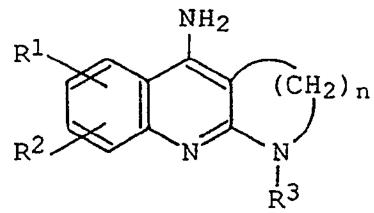

(I)

wherein $R^1$ and $R^2$ each represent a hydrogen atom, a halogen atom, a lower alkyl group, a trifluoromethyl group, a hydroxyl group, a lower alkoxy group, a lower alkanoyloxy group, a nitro group, an amino group or a lower alkanoylamino group ; $R^3$ represents a hydrogen atom, an alkyl group having 1 to 15 carbon atoms, a cycloalkyl group, an aralkyl group having 7 to 15 carbon atoms which may be substituted by a halogen atom, a lower alkyl group or a lower alkoxy group, an alkanoyl group having 2 to 15 carbon atoms, or a benzoyl group which may be substituted by a halogen atom, a lower alkyl group, a lower alkoxy group, a nitro group, a hydroxy group or an amino group ; and n represents an integer of 2 to 5, and an acid addition salt thereof.

2. The quinoline compound according to Claim 1, wherein n is 2 to 4.

3. The quinoline compound according to Claim 1, wherein n is 2 and $R^3$ is a hydrogen atom, an alkyl group having 1 to 15 carbon atoms, a cycloalkyl group, or an aralkyl group having 7 to 15 carbon atoms which may be substituted by a halogen atom, a lower alkyl group or a lower alkoxy group.

4. The quinoline compound according to Claim 1, wherein n is 2 and $R^3$ is an alkanoyl group having 2 to 15 carbon atoms, or a benzyl group which may be substituted by a halogen atom, a lower alkyl group or a lower alkoxy group.

5. The quinoline compound according to Claim 1, wherein n is 3 and $R^3$ is a hydrogen atom, an alkyl group having 1 to 15 carbon atoms, a cycloalkyl group, or an aralkyl group having 7 to 15 carbon atoms which may be substituted by a halogen atom, a lower alkyl group or a lower alkoxy group.

6. The quinoline compound according to Claim 1, wherein n is 3 and $R^3$ is an alkanoyl group having 2 to 15 carbon atoms, or a benzyl group which may be substituted by a halogen atom, a lower alkyl group or a lower alkoxy group.

7. The quinoline compound according to Claim 1, wherein n is 4 and $R^3$ is a hydrogen atom, an alkyl group having 1 to 15 carbon atoms, a cycloalkyl group, or an aralkyl group having 7 to 15 carbon atoms which may be substituted by a halogen atom, a lower alkyl group or a lower alkoxy group.

8. The quinoline compound according to Claim 1, wherein n is 4 and $R^3$ is an alkanoyl group having 2 to 15 carbon atoms, or a benzyl group which may be substituted by a halogen atom, a lower alkyl group or a lower alkoxy group.

9. The compound according to Claim 1, wherein the halogen atom is a fluorine atom, a chlorine atom, a bromine atom or an iodine atom.

10. The compound according to Claim 9, wherein the lower alkyl group of $R^1$ or $R^2$ is a straight or branched alkyl group selected from a methyl group, an ethyl group, a n-propyl group, an isopropyl group, a n-butyl group, a sec-butyl group, an isobutyl group and a t-butyl group.

11. The compound according to Claim 10, wherein the alkoxy group of $R^1$ or $R^2$ is an alkoxy group having 1 to 4 carbon atoms selected from a methoxy group, an ethoxy group, a n-propoxy group, an isopropoxy group, a n-butoxy group, a sec-butoxy group and an isobutoxy group.

12. The compound according to Claim 11, wherein the alkanoyloxy group of $R^1$ or $R^2$ is an alkanoyloxy group having 1 to 4 carbon atoms selected from an acetoxy group, a n-propanoyloxy group, a n-butanoyloxy group and an isobutanoyloxy group.

13. The compound according to Claim 12, wherein the alkanoylamino group of $R^1$ or $R^2$ is an alkanoylamino group having 1 to 4 carbon atoms selected from acetylamino group, a n-propanoylamino group, a n-butanoylamino group and an isobutanoylamino group.

14. The compound according to Claim 13, wherein the alkyl group having 1 to 15 carbon atoms of $R^3$ is a straight or branched alkyl group selected from a methyl group, an ethyl group, a n-propyl group, an isopropyl group, a n-butyl group, an isobutyl group, a sec-butyl group, a n-pentyl group, an isopentyl group, a neo-pentyl group, a hexyl group, a heptyl group, an octyl group, a decyl group, a dodecyl group and a pentadecyl group.

15. The compound according to Claim 14, wherein the cycloalkyl group of $R^3$ is a cycloalkyl group having 3 to 10 carbon atoms selected from a cyclopropyl group, a cyclobutyl group, a cyclopentyl group, a cyclohexyl group, a cycloheptyl group, a cyclooctyl group and a cyclodecyl group.

16. The compound according to Claim 15, wherein the aralkyl group of $R^3$ is selected from a benzyl group, a 4-chlorobenzyl group, a 3-chlorobenzyl group, a 4-mehtoxybenzyl group, a 3-mehtoxybenzyl group, a 3,4-dimethoxybenzyl group, a 4-ethoxybenzyl group, a 4-methylbenzyl group, a 2-phenylethyl group, a 2-(4-methoxyphenyl) ethyl group, a 2-(4-fluorophenyl) ethyl group, a 2-(4-methylphenyl) ethyl group, a 3-phenylpropyl group, a 3-(4-fluorophenyl) propyl group, a 4-phenylbutyl group and a 4-(4-methoxyphenyl-)butyl group.

17. The compound according to Claim 16, wherein the alkanoyl group of $R^3$ is a straight or branched alkanoyl group selected from an acetyl group, a n-propanoyl group, a n-butanoyl group, an isobutanoyl group, a n-pentanoyl group, an isopentaonoyl group, a n-hexanoyl group, a n-heptanoyl group, a n-octanoyl group, a n-decanoyl group and a n-dodecanoyl group.

18. The compound according to Claim 17, wherein the benzoyl group of $R^3$ is selected from a benzoyl group, a 4-chlorobenzoyl group, a 3-chlorobenzoyl group, a 2-chlorobenzoyl group, a 4-methylbenzoyl group, a 4-methoxybenzoyl group, a 3-methoxybenzoyl group, a 4-nitrobenzoyl group, a 4-aminobenzoyl group and a 4-hydroxybenzoyl group.

19. The compound according to Claim 18, wherein said $R^1$ or $R^2$ is a nitro group.

20. The compound according to Claim 1, wherein the acid addition salt of the compound is a salt with hydrochloric acid, hydrobromic acid, sulfuric acid, phosphoric acid, maleic acid, fumaric acid, oxalic acid or paratoluenesulfonic acid.

21. The compound according to Claim 1, wherein the compound is selected from 4-amino-1-methyl-2,3-dihydro-1H-pyrrolo[2,3-b]quinoline, 5-amino-1-benzyl-1,2,3,4-tetrahydrobenzo[b] [1,8]naphthylidine·hydrochloride 2/3 hydrate, 6-amino-1-dodecyl-7-fluoro-2,3,4,5-tetrahydro-1H-azepino[2,3-b]quinoline, 4-amino-5-fluoro-1-methyl-2,3-dihydro-1H-pyrrolo[2,3-b]quinoline, 4-amino-5-chloro-1-methyl-2,3-dihydro-1H-pyrrolo[2,3-b]quinoline, 4-amino-1-benzyl-2,3-dihydro-1H-pyrrolo[2,3-b]quinoline, 5-amino-1-benzyl-6-fluoro-1,2,3,4-tetrahydrobenzo[b] [1,8]naphthylidine, 5-amino-1-benzyl-6-fluoro-1,2,3,4-tetrahydrobenzo[b] [1,8]naphthylidine, 5-amino-1-benzyl-6-chloro-1,2,3,4-tetrahydrobenzo[b] [1,8]naphthylidine, 5-amino-8-chloro-1-(m-methoxybenzyl)-1,2,3,4-tetrahydrobenzo[b] [1,8]naphthylidine, 6-amino-1-benzyl-2,3,4,5-tetrahydro-1H-azepino[2,3-b]quinoline·hydrochloride, 6-amino-1-benzyl-7-chloro-2,3,4,5-tetrahydro-1H-azepino[2,3-b]quinoline·hydrochloride, 6-amino-1-methyl-2,3,4,5-tetrahydro-1H-azepino[2,3-b]quinoline, 6-amino-7-fluoro-1-methyl-2,3,4,5-tetrahydro-1H-azepino[2,3-b]quinoline, 6-amino-7-chloro-1-methyl-2,3,4,5-tetrahydro-1H-azepino[2,3-b]quinoline, 6-amino-1-(3-phenylpropyl)-2,3,4,5-tetrahydro -1H-azepino[2,3-b]quinoline·hydrochloride, 6-amino-1-heptyl-9-methoxy-2,3,4,5-tetrahydro-1H-azepino[2,3-b]quinoline, 6-amino-1-heptyl-2,3,4,5-tetrahydro-1H-azepino[2,3-b]quinoline hydrochloride·1/2 hydrate, 5-amino-6-chloro-1-(m-methoxybenzyl)-1,2,3,4-tetrahydrobenzo[b] [1,8]naphthylidine, 5-amino-1,2,3,4-tetrahydrobenzo[b] [1,8]naphthylidine·hydrochloride, 5-amino-6-fluoro-1,2,3,4-tetrahydrobenzo[d] [1,8]naphthylidine, 5-amino-6-chloro-1,2,3,4-tetrahydrobenzo[b] [1,8]naphthylidine, 4-amino-2,3-dihydro-1H-pyrrolo[2,3-b]quinoline·hydrobromide, 6-amino-2,3,4,5-tetrahydro-1H-azepino[2,3-b]quinoline·hydrochloride, 1-acetyl-5-amino-6-fluoro-1,2,3,4-tetrahydrobenzo[b] [1,8]naphthylidine, 5-amino-1-octanoyl-1,2,3,4-tetrahydrobenzo[b] [1,8]naphthylidine, 5-amino-1-octanoyl-1,2,3,4-tetrahydrobenzo[b] [1,8]naphthylidine·hydrochloride·1/2 hydrate, 5-amino-1-benzoyl-1,2,3,4-tetrahydrobenzo[b] [1,8]naphthylidine, 4-amino-1-isoamyl-2,3-dihydro-1H-pyrrolo[2,3-b]quinoline·hydrochloride, 4-amino-6-chloro-1-cyclohexyl-2,3-dihydro-1H-pyrrolo[2,3-b]quinoline, 4-amino-6-nitro-1-benzyl-2,3-dihydro-1H-pyrrolo[2,3-b]quinoline, 5-amino-7-nitro-1-benzyl-1,2,3,4-tetrahydrobenzo[b] [1,8]naphthylidine, 5-amino-8-chloro-1,2,3,4-tetrahydrobenzo[b] [1,8]naphthylidine, 4-amino-1-phenylethyl-2,3-dihydro-1H-pyrrolo[2,3-b]quinoline·monohydrate, 4-amino-5-fluoro-1-isoamyl-2,3-dihydro-1H-pyrrolo[2,3-b]quinoline·trihydrate and 5-amino-8-chloro-1-(m-hydroxybenzyl)-1,2,3,4-tetrahydrobenzo[b][1,8]naphthylidine.